(19) Europäisches Patentamt
European Patent Office
Office européen des brevets

(11) **EP 3 785 755 A1**

(12) **EUROPEAN PATENT APPLICATION**

(43) Date of publication:
**03.03.2021 Bulletin 2021/09**

(51) Int Cl.:
**A61M 16/16** (2006.01)    **A61M 16/00** (2006.01)

(21) Application number: **19194097.2**

(22) Date of filing: **28.08.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **LOUWSMA, Hendrik Klaas**
  **5656 AE Eindhoven (NL)**
• **STOLK, Theodor**
  **5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **HUMIDIFYING A GAS FLOW STREAM**

(57)    An apparatus (100) for humidifying a gas flow stream (110) is described. The apparatus comprises a plurality of humidification chambers (114, 116) connected in a sequence to allow the gas flow stream to flow sequentially through the plurality of humidification chambers. Each humidification chamber is configured to contain a liquid (124) over which the gas flow stream can flow. At least one humidification chamber comprises a nozzle (118) that forms the gas flow stream into a gas jet (120) that impinges on a surface (122) of the liquid (124) in each humidification chamber. The humidity level of the gas increases as the gas flow stream flows sequentially through the plurality of humidification chambers.

Figure 1a

EP 3 785 755 A1

Printed by Jouve, 75001 PARIS (FR)

**Description**

TECHNICAL FIELD OF THE INVENTION

Embodiments relate to apparatus, systems and methods for humidifying a gas which may be supplied, for example, to a patient or other subject for inhalation.

BACKGROUND OF THE INVENTION

For some ventilator systems and continuous positive airway pressure (CPAP) systems, humidified air may be supplied to a patient or other subject for inhalation. Humidifiers used in non-portable ventilator systems and CPAP systems may use a dedicated heater such as a hot plate to evaporate water to obtain a desired humidity level. However, there is a trend for these systems to be used at a patient's home. To facilitate use of such systems at home, end-users may consider it desirable for the system to be sufficiently compact and light so as to be portable.

Further, it may be desirable to ensure that the systems may operate with minimal power consumption to permit use of such systems where there is no or a restricted power supply. Batteries may be used to supply power to such systems although batteries contribute to the size and weight of the system, which may in turn affect the portability of such battery-power systems. However, the limited power supplied by batteries may pose additional challenges for humidifiers since additional power may be expended to provide heat for evaporating water.

Ventilator systems and CPAP systems may be equipped with a cold passover humidifier for humidifying a gas which may be supplied to a patient or other subject for inhalation. Cold passover humidification involves passing a gas such as air over a water surface to evaporate water without the need to provide a dedicated heater for aiding the evaporation process. Accordingly, cold passover humidification may be considered to be a relatively simple and inexpensive technique for humidifying a gas in comparison to other humidifying techniques.

However, the lack of a dedicated heater in cold passover humidifiers may limit the degree to which the gas can become humidified due to a low rate of evaporation of water compared with humidifiers equipped with a dedicated heater. The evaporation process is limited by the amount of heat the cold passover humidifier can extract from its surrounding environment. Typically, around 4 to 5 W of heat can be extracted from the environment in some cold passover humidifiers. Further, the cold passover humidification process may reduce the temperature of the gas being humidified since the evaporation process extracts heat from the water, which cools down during operation of the humidifier. The colder water also cools down the air as it flows over the water. At equilibrium, the water may be cooled to around 12 to 14 °C when the system is operating at room temperature with the humidified air leaving the system several degrees cooler than room temperature. This cooling effect may be undesirable for a patient inhaling the cooled humidified gas.

Accordingly, an object is to improve the humidification performance of a cold passover humidification system. Another object is to improve the comfort to a subject of a humidified gas flow provided by a cold passover humidification system.

SUMMARY OF THE INVENTION

Embodiments described herein relate to improving the humidification performance of a cold passover humidification system, which may supply humidified gas to a patient or other subject for inhalation. Embodiments described herein also relate to improving the comfort to a subject of a humidified gas flow provided by a cold passover humidification system. The inventors have identified apparatus and a system for humidifying a gas and a corresponding method which obviate one or more problems with cold passover humidification systems while maintaining or improving one or more benefits of such humidification systems.

In an embodiment, an apparatus for humidifying a gas flow stream is described. The apparatus comprises a plurality of humidification chambers. The humidification chambers are configured to be connected in a sequence to allow the gas flow stream to flow sequentially through the plurality of humidification chambers. Each humidification chamber is configured to contain a liquid over which the gas flow stream can flow. At least one of the humidification chambers comprises a nozzle configured to form the gas flow stream into a gas jet that impinges on a surface of the liquid in the humidification chamber.

In another embodiment, a system for humidifying a gas flow stream is described. The system comprises a source device for generating the gas flow stream; an apparatus according to any embodiment described herein; and an interface for delivering a humidified gas flow stream from the apparatus to a subject.

In another embodiment, a method of humidifying a gas flow stream is described. The method comprises passing a gas flow stream sequentially through a plurality of humidification chambers connected in a sequence. Each humidification chamber is configured to contain a liquid over which the gas flow stream can flow. The gas flow stream is formed into a gas jet in at least one of the plurality of humidification chambers such that the gas jet impinges on a surface of the liquid in the humidification chamber.

Embodiments described herein may provide efficient and/or cost effective humidification of a gas and/or may provide more comfort to a subject of a humidified gas flow provided by a cold passover humidification system. For example, the embodiments described herein may be capable of improving the humidification performance of a cold passover humidification system and/or improve the comfort to a subject of a humidified gas flow provided by a cold passover humidification system.

These and other aspects of the invention will be apparent from and elucidated with reference to the embodiment(s) described hereinafter.

BRIEF DESCRIPTION OF DRAWINGS

Exemplary embodiments of the invention will now be described, by way of example only, with reference to the following drawings, in which:

> Figure 1a is a schematic drawing of an apparatus for humidifying a gas flow stream according to an embodiment;
> Figure 1b is an expanded view of a gas jet depicted by Figure 1a;
> Figure 2 is a graph depicting a comparison in the humidification performance of an apparatus according to an embodiment with two comparative example apparatus;
> Figure 3 is a schematic drawing of another gas jet according to an embodiment;
> Figure 4 is a schematic drawing of another apparatus for humidifying a gas flow stream according to an embodiment;
> Figure 5 is a graph depicting a simulation of temperature changes during operation of an apparatus according to an embodiment; and
> Figure 6 refers to a method of humidifying a gas flow stream according to an embodiment.

DETAILED DESCRIPTION OF THE EMBODIMENTS

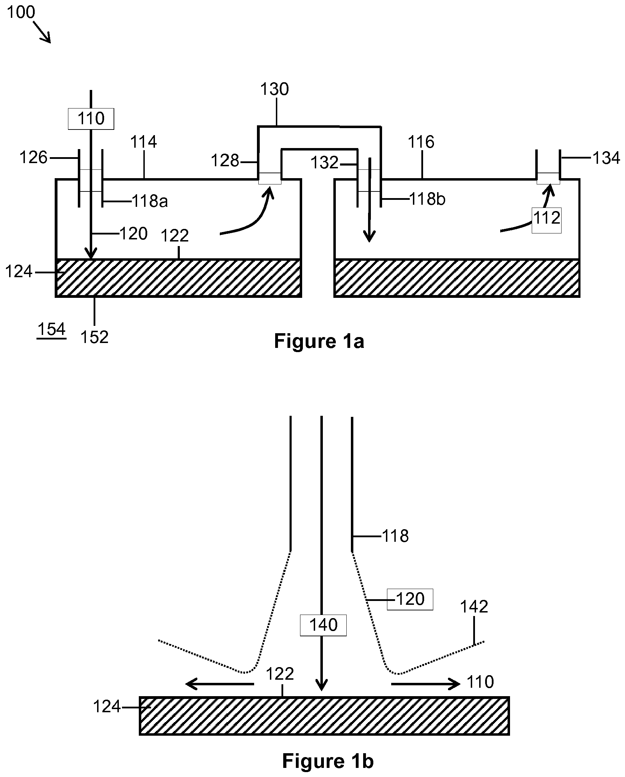

Figure 1a schematically depicts an apparatus 100 for humidifying a gas flow stream 110 to provide a humidified gas flow stream 112, which may be used as therapy gas for a patient or other subject. The apparatus 100 is a cold passover humidification apparatus, and thus does not include any active (i.e. powered) heating elements for heating liquid in the apparatus 100 or the gas flow stream 110. The apparatus 100 comprises two humidification chambers 114, 116 that are connected in a sequence to allow the gas flow stream 110 to flow sequentially through the two humidification chambers 114, 116. Each humidification chamber 114, 116 is configured to contain a liquid 124 such as water or distilled water over which the gas flow stream 110 can flow. The gas flow stream 110 enters a first humidification chamber 114 of the two humidification chambers 114, 116 via an inlet 126 of the first humidification chamber 114. The inlet 126 may also be the main gas flow stream inlet for the apparatus 100. The gas flow stream 110 passes through the first humidification chamber 114 to increase a humidity level of the gas flow stream 110 as it passes over the liquid 124 in the humidification chamber 114. The gas flow stream 110 leaves the first humidification chamber 114 via an outlet 128 of the first humidification chamber 114 to enter a second (final) humidification chamber 116 of the two humidification chambers 114, 116 via an inlet 132 of the final humidification chamber 116. The outlet 128 of the first humidification chamber 114 and the inlet 132 of the final humidification chamber 116 may be connected by any suitable means, for example via a hose 130 or other flexible coupling, or for example via a rigid coupling. Alternatively, although the two humidification chambers 114, 116 are shown in Figure 1 as being physically separate structures, in other implementations the two humidification chambers 114, 116 can be separate chambers defined within a single housing, in which case the outlet 128 of the first humidification chamber 114 may be directly connected to or integral with the inlet 132 of the final humidification chamber 116. The gas flow stream 110 passes through the final humidification chamber 116 to further increase a humidity level of the gas flow stream 110 as it passes over the liquid 124 in the final humidification chamber 116. The humidified gas flow stream 112 then leaves the final humidification chamber 116 via an outlet 134 of the final humidification chamber 116. The outlet 134 may also be the main gas flow stream outlet for the apparatus 100.

The inlet of the apparatus 100 (e.g. the inlet 126 of the first humidification chamber 114) can be configured to be coupled to a source of the gas flow stream 110, such as a ventilator or a continuous positive airway pressure (CPAP) device, that provides a flow of gas that is to be inhaled by a patient or other subject.

This humidified gas flow stream 112 output by the outlet of the apparatus 100 (e.g. the outlet 134 of the final humidification chamber 116) can be provided to a patient or other subject for inhalation. The humidified gas flow stream 112 can be provided to the patient or other subject via a hose or other flexible coupling between the outlet of the apparatus 100 and a patient interface (not shown in Figure 1a), such as a full face mask, a partial face mask, a nasal tube, a mouthpiece, etc.

Preferably, each of the humidification chambers 114, 116 comprise a nozzle 118a,b (also referred to herein as a nozzle 118) at or associated with their respective inlets 126, 132 configured to form the gas flow stream 110 into a gas jet 120 and direct the gas jet 120 to impinge on a surface 122 of the liquid 124 in the respective humidification chamber 114, 116. However, in other embodiments, at least one of the humidification chambers 114, 116 may comprise such a nozzle 118 whereas at least one other of the humidification chambers 114, 116 may comprise a different type of opening into the humidification chamber that does not form the gas flow stream 110 into a gas 'jet'.

The inventors have recognized that the humidification performance of the apparatus 100 may be improved by forming a gas jet 120 in at least one of the humidification chambers 114, 116. Further, the combination of the plurality of humidification chambers 114, 116 and the at least one gas jet 120 may together provide a particularly improved performance for humidifying the gas flow stream 110 as compared with certain other types of humidification systems.

Figure 1b is an expanded view of part of the apparatus 100 and schematically depicts the effect of the gas jet 120 in more detail. The nozzle 118 is configured to direct the gas jet 120 towards the surface 122 of the liquid 124 such that, after impinging on the surface 122, the gas flow stream 110 flows radially outwards from the point of impingement over the surface 122 of the liquid 124 to disrupt a boundary diffusion layer above the surface 122, to promote humidification of the gas flow stream 110. The boundary diffusion layer comprises vapor from the liquid 124, which when disrupted by the gas flow stream 110, is carried by the gas flow stream 110 to thereby increase the humidity level of the gas flow stream 110. In some embodiments, the radial flow from the point of impingement is in the form of a gas wall jet 142.

The nozzle 118 is configured such that the gas jet 120 impinges perpendicularly or substantially perpendicularly on the surface 122. In this embodiment, the nozzle 118 is configured to direct the gas jet 120 towards the surface 122 of the liquid 124 such that a core 140 of the gas jet 120 is oriented perpendicular or substantially perpendicular to the surface 122. The inventors have found that directing the gas jet 120 towards the surface 122 in this manner provides a specified performance level in terms of promoting humidification of the gas flow stream 110.

The geometry of certain components of the apparatus 100, as well as the desired gas flow stream properties may affect the properties of the gas jet 120. For example, in a medical respiratory application such as ventilation or CPAP, the gas flow stream 110 may be delivered at a rate of $Q = 0.25$ to $2.5$ liters per second. An expression for the gas flow rate, $Q$, through a nozzle 118 with a circular exit is $Q = \frac{1}{4}\pi v d^2$. For an example apparatus 100, the velocity, $v$, of the gas jet 120 at the exit of the nozzle 118 may be in the range 4 to 24 meters per second and the diameter, $d$, at the exit of the nozzle 118 may be in the range 0.4 to 2.4 cm. Additionally, the vertical distance (i.e., height) between the nozzle 118 exit and the surface 122 may be in the range 2 to 12 cm. A nozzle 118 with a larger diameter exit produces a gas jet 120 with a lower velocity than that of a nozzle 118 with a relatively smaller diameter exit. The gas flow rate is also determined by the pressure (i.e., head) delivered by a particular gas source and the flow resistance of the nozzle 118.

The inventors have also recognized that by providing a plurality of humidification chambers 114, 116 (i.e., two or more humidification chambers), it may be possible to improve the humidification performance of the gas flow stream 110 by increasing the humidity of the gas flow stream 110 to a desired level of humidity without having to employ an external heat source such as may be used in certain other types of humidification systems, thereby reducing the cost of operation and/or complexity of the apparatus 100 compared with certain cold passover humidification systems. As the gas flow stream 110 passes through the first humidification chamber 114 over the surface 122 of the liquid 124, some of the liquid 124 evaporates and thereby increases a humidity level of the gas. This humidification (evaporation) process uses heat energy, and so the evaporation decreases the temperature of the liquid 124. In typical ambient (room) temperatures, after the apparatus 100 has been used for some time (e.g. several minutes) the temperature of the liquid 124 will be below that of the incoming gas flow stream 110, and so the cooler liquid 124 will in turn cause a decrease in temperature of the gas flow stream 110 measured at the outlet 128. Thus, the gas that leaves the first humidification chamber 114 has a higher level of humidity, but lower temperature, than the gas that enters the first humidification chamber 114.

The inventors have also found that by providing a plurality of humidification chambers 114, 116, and passing the gas flow stream 110 through the humidification chambers 114, 116 in sequence, both the humidity level and the temperature of the gas that has previously passed through the first humidification chamber 114 can be increased, thereby improving the comfort to a subject of the humidified gas flow stream 112 compared with certain existing cold passover humidification systems. For example, while the temperature of the liquid 124 in the final humidification chamber 116 may also decrease due to the humidification process occurring in that chamber 116 (i.e. heat is lost from the liquid 124 in the final humidification chamber 116 due to evaporation), the rate of evaporation in the final humidification chamber 116 will be lower than in the first humidification chamber 114, and so, once a steady-state has been achieved in the apparatus 100, the temperature of the liquid 124 in the final humidification chamber 116 will be higher than the temperature of the liquid 124 in the first humidification chamber 114. In fact, the inventors have found that the temperature of the liquid 124 in the final humidification chamber 116 may be higher than the temperature of the humidified gas flow stream 110 received at the inlet 132 of the final humidification chamber 116, and so the gas passing through the final humidification chamber 114 may be heated by the liquid 124 therein. While the temperature of the humidified gas flow 112 at the outlet 134 will still be lower than the temperature of the gas flow 110 received at the inlet 126 providing there are no ambient temperature shocks, the increase in temperature may be beneficial to the patient or other subject that subsequently receives the humidified gas flow stream 112. However, if an ambient temperature shock occurs, there may be circumstances where the temperature of the humidified gas flow stream 112 at the outlet 134 is temporarily higher than the temperature of the gas flow stream 110 received at the inlet 126. For example, if a user moves the apparatus 100 from an environment with an ambient room temperature to another environment with a temperature (for example, 0 °C) that is lower than the temperature of the liquid 124, the gas flow stream 110 may be temporarily heated by the liquid 124 until an equilibrium state is reached after the liquid 124 has been cooled by the gas flow stream 110 from the lower-temperature environment.

The improvement of humidification performance may be achieved through passive use of the apparatus 100. In other similar words, the gas flows through the apparatus 100 simply due to the pressure of the gas flow from a source device such as a separate gas pump device (not shown), and there are no active components in the apparatus 100. For example, in this embodiment, the apparatus 100 has no dedicated heater or pump to push the gas through the humidification chambers 114, 116. Thus, the apparatus 100 may be used in scenarios where there is limited or no power available for humidifying a gas but a source device is present and available for generating a gas flow which can be humidified by the apparatus 100.

The inventors have also found that by thermally isolating the two humidification chambers 114, 116 so as to substantially prevent conduction of heat therebetween, the temperature of the liquid 124 in the final humidification chamber 116 may be higher than if the humidification chambers 114, 116 were separated by a material that is substantially conductive to heat (as the lower-temperature liquid 124 in the first humidification chamber 114 would act to further cool the liquid 124 in the final humidification chamber 116). In Figure 1a, the two humidification chambers 114, 116 are thermally isolated by air since the humidification chambers 114, 116 are physically separated from each other. However, in other examples, the humidification chambers 114, 116 may be physically connected to each other and may have a dividing wall with heat insulating properties that substantially thermally isolates the humidification chambers from each other.

During operation of the apparatus 100 (i.e. as a gas flow stream 110 is passed through the apparatus 100), providing the temperature of the environment around the humidification chambers 114, 116 is higher than that of the liquid 124 in the humidification chambers 114, 116, the environment (e.g. ambient air) can help to heat the liquid 124, which may aid the humidification process by helping to reduce the cooling of the liquid 124. Therefore, the humidification chambers 114, 116 may each comprise a heat conductive wall 152 to facilitate heat being conducted from an external environment 154 of the humidification chambers 114, 116 into the humidification chambers 114, 116 to heat the liquid 124 therein.

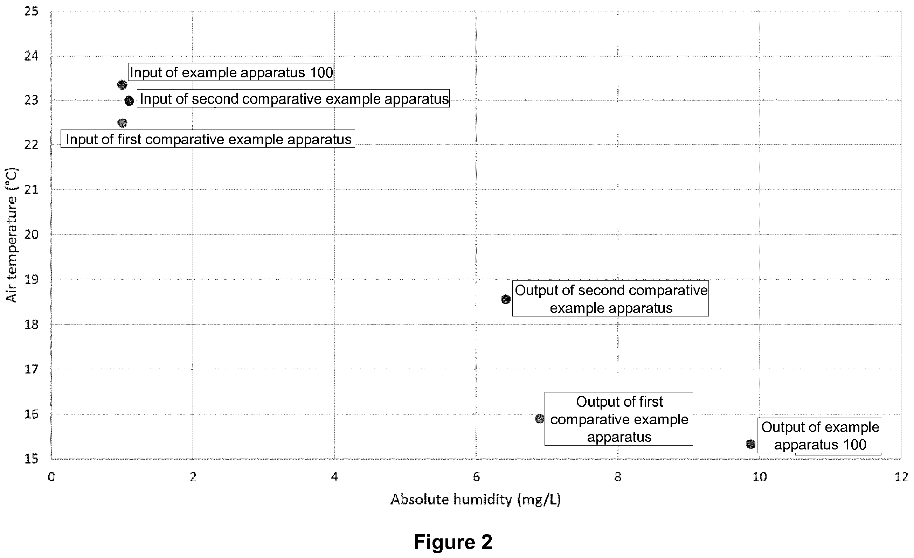

Figure 2 is a graph depicting experimental data obtained by comparing the performance of an example apparatus 100 comprising two humidification chambers (i.e., first and final humidification chambers 114, 116) with the performance of first and second comparative example apparatus. Each comparative example apparatus comprised a single humidification chamber. The first comparative example apparatus comprised the same type of humidification chamber as the example apparatus 100 (e.g., a first humidification chamber 114) and a nozzle 118 for forming a jet 120 that impinges on the surface of the liquid as described previously. The first comparative example apparatus contained half the volume of liquid compared with the total volume of liquid (i.e., 600 ml) contained within the example apparatus 100. The second comparative example apparatus comprised a different type of humidification chamber where the airflow therein is split into multiple parallel flows over the surface of the liquid contained therein. The second comparative example apparatus contained the same volume of liquid as the example apparatus 100 but the total surface area of the liquid contained in the apparatus 100 was 74% that of the second comparative example apparatus.

The x-axis of the graph represents the absolute humidity in mg/l of the air (i.e., the gas flow stream) at the indicated input or output of the apparatus 100 and the two comparative example apparatus. The y-axis of the graph represents the temperature in °C of the air supplied at the input or delivered from the output of the apparatus 100 and the two comparative example apparatus. The dots at the upper left-hand side of the graph refer to experimental data indicating the input state of the air (i.e., the temperature and absolute humidity) and the other dots towards the right-hand side of the figure refer to experimental data indicating the output state of the air, after being fed through the indicated apparatus 100 or comparative example apparatus.

The data for the graph was obtained by operating the example apparatus 100 and the comparative example apparatus with a gas (air) flow rate of 0.75 liters per second for around 1 hour at an ambient temperature of around 23 °C (as shown by the dots at the top left-hand side of the graph). Operation of the first comparative example apparatus under these conditions increased the absolute humidity of the gas flow stream 110 from around 1 mg/l to around 6.5-7 mg/l and decreased the air temperature at the output to around 16 °C. Operation of the second comparative example humidification apparatus under approximately the same conditions resulted in an increase of the absolute humidity of a gas flow stream from around 1 mg/l to around 6-6.5 mg/l and decreased the air temperature at the output to around 18.5 °C. The addition of the final humidification chamber 116 of the example apparatus 100 resulted in a further increase in the absolute humidity level of the gas flow stream 110 compared with the absolute humidity produced by first comparative example apparatus. In this regard, the absolutely humidity of the air at the output of the example apparatus 100 was around 10 mg/l, which represents an approximate 50% increase in the humidity level compared with the humidity for first and second comparative example apparatus. A further decrease in air temperature was observed at the output of the example apparatus 100 to around 15.5 °C compared with the air temperature (around 16 °C) at the output of the first comparative example apparatus. Thus, the addition of the second (final) humidification chamber 116 may provide a relatively effective but simple way to increase the humidity level of the gas flow stream 110.

In another example operation of the apparatus 100, a gas flow rate of 0.5 liters per second provided an evaporation power of around 8 W where the external environment contributed around 6 W and the gas flow contributed around 2 W to the evaporation power in an equilibrium state (i.e., where the liquid itself delivers no heat to the evaporation power except during initial, non-equilibrium, operation of the apparatus 100).

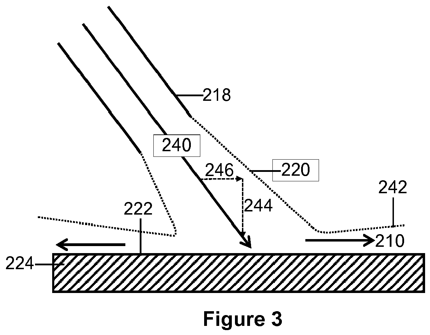

Figure 3 schematically depicts another nozzle 218 configuration where reference signs for features corresponding to similar features in Figure 1 are incremented by 100. Instead of being oriented perpendicular or substantially perpendicular to the surface 222 of the liquid 224, the nozzle 218 is oriented at an angle to the perpendicular such that a core 240 of a gas jet 220 formed by the nozzle 218 strikes the surface 222 at an angle (i.e., a "strike angle") to the vertical. Similar to the previous example, after impinging on the surface 222, the gas flow stream 210 flows outwards from the point of impingement over the surface 222 of the liquid 224 to disrupt a boundary diffusion layer above the surface 222, to promote humidification of the gas flow stream 210. In this embodiment, the nozzle 218 is configured to direct the gas jet 220 towards the surface 222 of the liquid 224 such that a first velocity component 244 of the core 240 in a direction perpendicular or substantially perpendicular to the surface 222 is greater than a second velocity component 246 of the core 240 of the gas jet 220 in a direction parallel to the surface 222. In other similar words, the resulting core 240 of the gas jet 220 is at a strike angle of less than 45 degrees to the vertical to the surface 222. A wall jet 242 formed after the point of impingement may take a different form to that of Figure 1 due to the different strike angle of the core 240. Accordingly, the strike angle of the core 240 may affect the humidification process. A strike angle of less than 10 degrees (i.e. perpendicular to the surface 222 or substantially perpendicular to the surface 222) may provide a specified level of performance in terms of humidifying the gas flow stream 210. However, various other factors such as gas flow rate, ambient temperature, apparatus geometry, among others may affect the humidification performance.

Although Figure 3 depicts a strike angle of less than 45 degrees to the vertical to the surface 222, in other embodiments, the strike angle may be equal to or more than 45 degrees to the vertical to the surface 222 providing the gas jet 220 impinges on the surface 222 (i.e. the strike angle should not be 90 degrees to the vertical to the surface 222 or substantially 90 degrees to the vertical to the surface 222). The selected strike angle may depend on certain factors such as the form of the gas flow stream 210 flowing over the surface 222, the distance between the nozzle 218 and the surface 222, the velocity of the gas jet 220 and the geometry of the apparatus, among other factors which may affect the humidification of the gas flow stream 210.

Returning again to Figure 1b, the vertical velocity component of the core 140 in the direction perpendicular or substantially perpendicular to the surface 122 is also greater than the horizontal velocity component in a direction parallel to the surface 122. Since the core 140 is oriented perpendicular or substantially perpendicular to the surface 122, the horizontal velocity component is substantially close to, or exactly, zero.

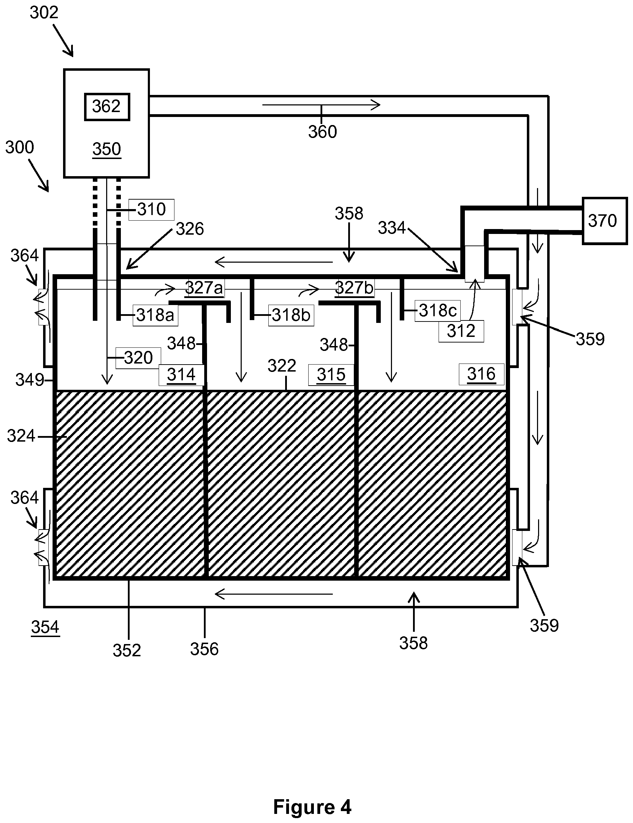

Apparatus described herein comprise a plurality of humidification chambers 114, 116. For example, the apparatus 100 of Figure 1 comprises two humidification chambers 114, 116. However, more than two humidification chambers may be provided, for example, as shown by Figure 4, which depicts another apparatus 300 according to an embodiment. Reference signs for features corresponding to similar features in Figure 1 are incremented by 200. The apparatus 300 comprises three humidification chambers 314, 315, 316 configured to be connected in a sequence to allow a gas flow stream 310 to flow sequentially through the humidification chambers 314, 315, 316. As before, the apparatus 300 comprises a first humidification chamber 314 and a final humidification chamber 316. Additionally, the apparatus 300 comprises an intermediate humidification chamber 315 disposed between the first and final humidification chambers 314, 316. Each humidification chamber 314, 315, 316 is configured to contain a liquid 324 over which the gas flow stream 310 can flow.

In this embodiment, the humidification chambers 314, 315, 316 are separated by two walls 348 extending vertically within a common housing 349 for the humidification chambers 314, 315, 316 where the two walls 348 divide the space in the common housing 349 into the three humidification chambers 314, 315, 316. The humidification chambers 314, 315, 316 are fluid-tight with respect to each other such that the liquids 324 in the humidification chambers 314, 315, 316 do not mix with each other. However, the first and intermediate humidification chambers 314, 315 are provided in fluid communication via a first chamber connector 327a disposed above the surface 322 of the liquid 324 in the first and intermediate humidification chambers 314, 315. Similarly, the intermediate and final humidification chambers 315, 316 are provided in fluid communication via a second chamber connector 327b disposed above the surface 322 of the liquid 324 in the intermediate and final humidification chambers 315, 316. The first and second chamber connectors 327a,b take the form of a passage to allow the gas flow stream 310 to flow therethrough from one humidification chamber 314, 315 to the subsequent humidification chamber 315, 316.

Accordingly, the first humidification chamber 314 in the sequence is configured to receive, at a gas inlet 326 of the apparatus 300, the gas flow stream 310 from a source device 350. The gas flow stream 310 is formed into a gas jet 320 by a nozzle 318a connected to the gas inlet 326. The gas flow stream 310 is passed through the first humidification chamber 314 to increase a humidity level of the gas flow stream 310 as it passes over the liquid 324 in the first humidification chamber 314.

The gas flow stream 310 then passes through the first chamber connector 327a, which comprises a nozzle 318b configured to form the gas flow stream 310 into a gas jet 320 so that the intermediate humidification chamber 315 receives the gas flow stream 310 that has previously passed through the first humidification chamber 314. The gas flow stream 310 passes through the intermediate humidification chamber 315, to increase the humidity of the gas flow stream 310 as it passes over the liquid 324 in the intermediate humidification chamber 315.

The gas flow stream 310 is then delivered, from the intermediate humidification chamber 315 towards the final humidification chamber 316 via the second chamber connector 327b. The second chamber connector 327b comprises a nozzle 318c configured to form the gas flow stream 310 into a gas jet 320 so that the final humidification chamber 316 receives the gas flow stream 310 that has previously passed sequentially through the first and intermediate humidification chamber 314, 315.

The final humidification chamber 316 in the sequence is configured to deliver the humidified gas flow stream 312 to a gas outlet 334 of the apparatus 300 after passing the gas flow stream 310 through the final humidification chamber 316 to further increase the humidity level of the gas flow stream 310 (that has previously passed sequentially through the first and intermediate humidification chambers 314, 315) as it passes over the liquid 324 in the final humidification chamber 316. As noted above with respect to the apparatus 100 shown in Figure 1, the humidified gas stream 312 from the gas outlet 334 may be provided to a patient or other subject via a patient interface.

It has previously been discussed in relation to Figure 1 that thermally isolating the two humidification chambers 114, 116 from each other may allow the temperature differential between the two humidification chambers 114, 116 to be maintained such that the gas flow stream 110 may be somewhat heated by the warmer liquid 124 in the final humidification chamber 116 after being initially cooled after passing through the first humidification chamber 114. The addition of the intermediate humidification chamber 315 may also improve this heating of the gas flow stream 310 such that the resulting humidified gas flow stream 312 may experience an increase in temperature compared with the temperature of the gas flow stream 310 that enters the final humidification chamber 316. Accordingly, the apparatus 300 of Figure 3 may improve the comfort to a subject of the humidified gas flow stream 312 due to its increased temperature.

In Figure 1 the humidification chambers 114, 116 are physically separated from each other such that air between the humidification chambers 114, 116 defines a thermally insulating portion. In Figure 4, the walls 348 each comprise a thermally insulating portion configured to substantially prevent conduction of heat between adjacent humidification chambers 314, 315, 316. Examples of walls 348 comprising thermally insulating portions include cavity walls, foam-based insulating walls, plastic walls, and the like. Although the walls 348 may be configured to thermally isolate the humidification chambers 314, 315, 316 from each other, even if the walls 348 were not considered to have good thermal isolating properties, a degree of thermal isolation may still be achieved by provision of the walls 348 to the extent that a temperature differential between the humidification chambers 314, 315, 316 may be maintained at thermal equilibrium while the gas flow stream 310 is being humidified by the apparatus 300.

As described previously, heat is conducted into the liquid 324 from an external environment 354 during operation of the apparatus 300. Accordingly, at least one of the plurality of humidification chambers 314, 315, 316 may comprise a heat conductive wall 352 to facilitate extraction of heat from an external environment 354 of the apparatus 300 into the humidification chamber 314, 315, 316 to heat the liquid 324 therein and thereby assist in the process of humidification. In this embodiment, the housing 349 comprises the heat conductive wall 352.

In some embodiments the apparatus 300 further comprises an additional housing 356 extending around at least part of one or more of the humidification chambers 314, 315, 316 and defining a passage 358 between the heat conductive wall 352 of those humidification chambers 314, 315, 316 and the additional housing 356. The passage 358 is provided with an exhaust gas inlet 359 for receiving an exhaust gas stream 360 from a source device 350 for the gas flow stream 310 to pass around an outside of the heat conductive wall 352 so that heat in the exhaust gas stream 360 conducts into the liquid 324 in the humidification chamber 314, 315, 316. In this embodiment, the source device 350 comprises a cooling device 362 configured to cool the source device 350, which heats up during operation as it delivers the gas flow stream 310 to the apparatus 300 due to operating pumps/fans in the source device 350 for providing the gas flow stream 310. The heat from the exhaust gas stream 360 may be conducted into the liquid 324 to aid the humidification of the gas flow stream 310. The temperature of the exhaust gas stream 360 is therefore likely to be lower as it exits the passage 358 via a vent 364 compared with the temperature of the exhaust gas stream 360 as it enters the passage 358 via the exhaust gas inlet 359.

The apparatus 300 and the source device 350 (such as a ventilator or CPAP system, etc.) may form part of a system 302 (e.g., a ventilation system or a CPAP system), which may also comprise an interface 370 (e.g., a patient interface such as a full face mask, a partial face mask, a nasal tube, a mouthpiece, etc.) for receiving the humidified gas flow stream 312 from the apparatus 300.

Although the system 302 is depicted as comprising the source device 350 and the interface 370, other systems may comprise any combination of these elements. For example, in an embodiment the system 302 could comprise the apparatus 300 and the source device 350. In another embodiment, the system 302 could comprise the apparatus 300 and the interface 370. In any of these embodiments, the apparatus 300 may be integrated with the other components to, for example, provide more efficient heat harvesting and/or less tubing connecting the various components.

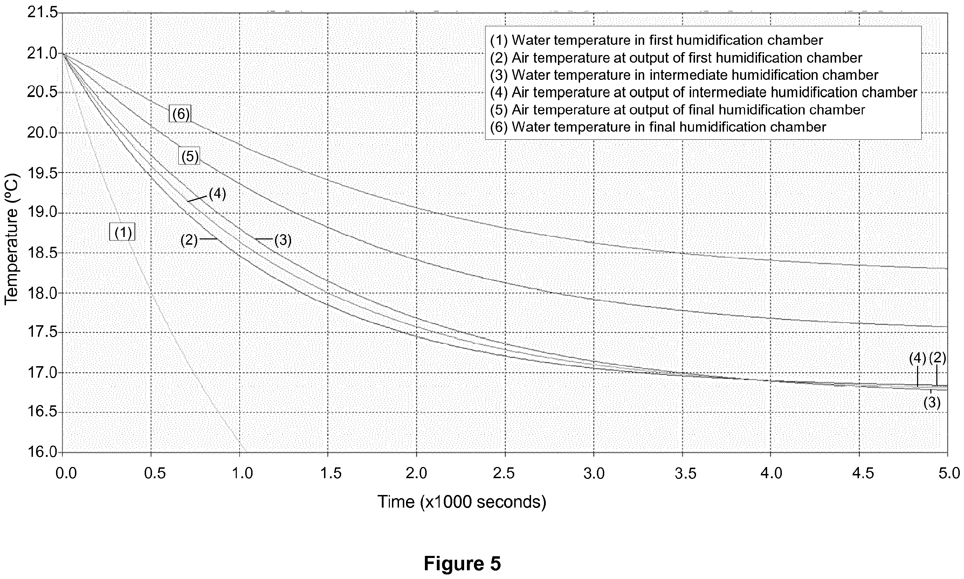

Figure 5 is a graph depicting an example simulation of an apparatus comprising three humidification chambers such as described above in relation to Figure 4 in which the following behavior was observed. At a starting temperature of 21 °C, the temperature of the liquid (i.e., water) in the first humidification chamber (line (1) in Figure 5) falls below 16 °C within around 17 minutes due to the humidification process. However, it has been determined that the temperature of the liquid in the first humidification chamber could fall to around 12 °C at a thermal equilibrium state, at which point, the energy for vaporizing the liquid is substantially extracted from the external environment rather than the latent heat of the liquid. As noted above, in an equilibrium state, the majority of the evaporation power is derived from the external environment (i.e., 6 W in the previous example) and the rest of the evaporation power is derived from the gas flow (i.e., 2 W in the previous example) with no contribution from the heat of the liquid itself except during initial, non-equilibrium, operation. As a result of the fall in temperature of the liquid in the first humidification chamber, the temperature of the gas flow stream flowing out of the first humidification chamber (line (2) in Figure 5) also falls to around 16.8 °C after over an hour. Similar behavior is observed for the temperature of the liquid (line (3) in Figure 5) and the resulting temperature of the gas flow stream (line (4) in Figure 5) in the intermediate humidification chamber. Although the fall in temperature is less rapid than for the gas flow stream in the first humidification chamber, at equilibrium, both the liquid and the gas flow stream in the intermediate humidification chamber also falls to a temperature of around 16.8 °C after over an hour. The third and final humidification chamber has the effect of increasing the temperature of the gas flow stream to around 17.6 °C (line (5) in Figure 5) at equilibrium since the temperature of the liquid in the final humidification chamber only falls to around 18.3 °C (line (6) in Figure 5) due to the reduced rate of evaporation in that chamber, thereby adding heat to the (cooler) gas flow stream as it passes through the final humidification chamber.

It has been determined that for an example operation of an apparatus comprising three humidification chambers such as described in relation to Figure 4, the first humidification chamber 314 may increase the humidity level by around 6 mg/l, the intermediate humidification chamber 315 may increase the humidity level by around 4 mg/l and the final humidification chamber 316 may increase the humidity level by around 2 mg/l. Accordingly, the total increase of humidity level may be around 12 mg/l for a three humidification chamber-based apparatus, which may represent a significant improvement in humidification performance compared with a single humidification chamber-based apparatus while maintaining the simplicity and cost benefits enjoyed by certain humidification systems such as cold passover humidification systems.

Although not shown in the figures, a liquid delivery system may be provided to replenish the liquid levels in the humidification chambers as the liquid levels deplete as the humidification process occurs. Since the liquid in each humidification chamber is isolated from each other, separate liquid inlets may be provided for each humidification chamber to deliver liquid into each humidification chamber and/or to ensure that the liquid in the humidification chambers does not mix, thereby maintaining a temperature differential between the humidification chambers. The liquid delivery system may be automatic or manual. For example, liquid may automatically be replenished from a separate reservoir in response to a reduction in the level of the liquid in one of more of the humidification chambers. In another example, a user may open the apparatus or otherwise access the one or more humidification chambers and put liquid into the chamber(s).

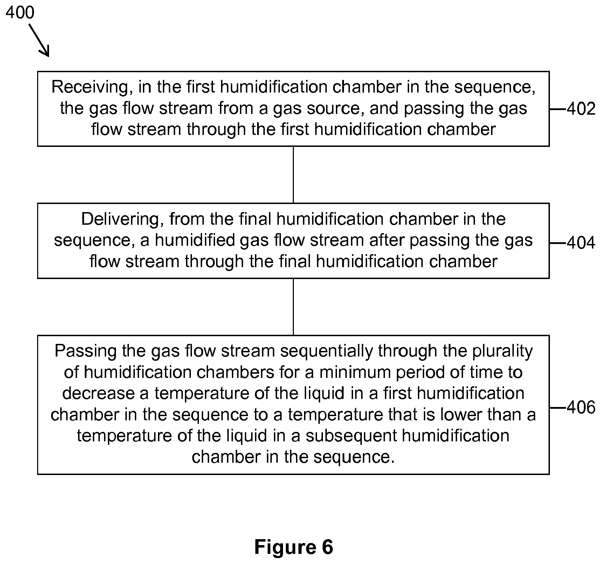

The functionality described in relation to the apparatus described herein may be implemented as a method. Figure 6 shows a method 400 which may be implemented by operation of the apparatus 100, 300. With initial reference to Figures 1 and 3, the method 400 comprises passing a gas flow stream 110 sequentially through a plurality of humidification chambers 114, 116 connected in a sequence. The gas flow stream 110 is formed into a gas jet 120 in at least one of the plurality of humidification chambers 114, 116 such that the gas jet 120 impinges on the surface 122 of the liquid 124 in the humidification chamber 114, 116. The method 400 comprises directing the gas jet 120, 220 towards the surface 122, 222 of the liquid 124, 224 such that a first velocity component 244 of the core 140, 240 of the gas jet 120, 220 in a direction perpendicular or substantially perpendicular to the surface 122, 222 is greater than a second velocity component 246 of the core 140, 240 of the gas jet 120, 220 in a direction parallel to the surface 122, 222.

With reference also to Figures 1 and 4, block 402 of the method 400 comprises receiving, in the first humidification chamber 114, 314 in the sequence, the gas flow stream 110, 310 from a source device 350, and passing the gas flow stream 110, 310 through the first humidification chamber 114, 314 to increase a humidity level of the gas flow stream 110, 310. The method 400 further comprises, at block 404, delivering, from the final humidification chamber 116, 316 in the sequence, a humidified gas flow stream 112, 312 after passing the gas flow stream 110, 310 through the final humidification chamber 116, 316 to further increase the humidity level of the gas flow stream 110, 310 that has previously passed through the first humidification chamber 114, 314.

The method 400 further comprises, at block 406, passing the gas flow stream 110, 310 sequentially through the plurality of humidification chambers 114, 116, 314, 315, 316 for a minimum period of time to decrease a temperature of the liquid in a first humidification chamber 114, 314 in the sequence to a temperature that is lower than a temperature of the liquid in a subsequent humidification chamber 116, 315, 316 in the sequence. Operating the apparatus 100, 300 for the minimum period of time (e.g., without any temperature shocks) results in the temperature of the gas flow stream 110, 310 exiting the first humidification chamber 114, 314 being decreased compared with the temperature of the gas flow stream 110, 310 entering the first humidification chamber 114, 314. Further, the temperature of the gas flow stream 110, 310 passing through the subsequent humidification chamber 116, 315, 316 is increased due to heat transfer from the liquid 124, 324 in the subsequent humidification chamber 116, 315, 316 to the gas flow stream 110, 310.

While the invention has been illustrated and described in detail in the drawings and foregoing description, such illustration and description are to be considered illustrative or exemplary and not restrictive; the invention is not limited to the disclosed embodiments. For example, embodiments described herein may supply humidified gas to be inhaled by a patient or other subject. However, the embodiments may be used for other humidification applications such as humidifying an environment. Although the embodiments refer generally to humidification, the liquid may comprise substances other than water, which may also be vaporized during the humidification process. Therefore, the embodiments may be capable of delivering such substances into the gas flow stream as well as humidifying the gas flow stream. For example, aromas or medicines could be added to or dissolved into the water and then evaporated during the humidification process. Further, a biocide or alcohol could be added to the water e.g., for disinfection of components that come into contact with the humidified gas flow stream. Further, a colorant could be added into the water e.g., for visualization purposes.

In another application of the principles described herein, the cooling effect experienced by at least the first humidification chamber 114, 314 due to the humidification process may be used for various cooling applications.

In the embodiment where the exhaust gas stream 360 from the source device 350 for the gas flow stream 310 passes around an outside of the heat conductive wall 352, heat in the exhaust gas stream 360 conducts into the liquid 324 in the humidification chamber 314, 315, 316. In an alternative embodiment, the heat from the source device 350 may be harvested by the liquid 324 by means of conduction. For example, the source device 350 may be provided in contact with the heat conductive wall 352 such that heat from the source device 350 may be conducted into the liquid 324.

One or more features described in one embodiment may be combined with or replace features described in another embodiment. For example, the apparatus 100 of Figure 1 may be modified based on features described in relation to the apparatus 300 of Figure 4, and vice versa. For example, the humidification chambers 114, 116 of Figure 1 may be provided as part of a common housing as depicted by the humidification chambers 314, 315, 316 of Figure 4. Correspondingly, the humidification chambers 314, 315, 316 of Figure 4 may be physically separated from each other such as shown by Figure 1. Corresponding features to enable the gas flow stream 110, 310 to flow sequentially through the humidification chambers 114, 116, 314, 315, 316 such as the hose 130 of Figure 1 or the chamber connectors 327a,b of Figure 4 may be appropriately modified to compensate for any modifications to the configuration of the humidification chambers 114, 116, 314, 315, 316. Further, the humidification chamber configurations shown in Figures 1 and 4 may be modified. For example, the humidification chambers 114, 116, 314, 315, 316 depicted in Figures 1 and 4 could be stacked vertically, or arranged in any appropriate way.

Although the embodiments described herein refer to a humidification process where evaporation of the liquid (e.g., comprising water) in the humidification chambers humidifies the gas flow stream, in other embodiments a different liquid (which may comprise a substance other than water) may be evaporated or vaporized. In such other embodiments, the evaporation or vaporization of the liquid may result in the vapor from the liquid being incorporated into the gas flow stream. Thus, any reference to a "humidification chamber" described herein may also refer to a "vaporization chamber" or "evaporation chamber". Further, any reference to an apparatus for humidifying a gas flow stream (or a corresponding method or system) may also refer to an apparatus (or a corresponding method or system) for incorporating vapor from a liquid in a vaporization chamber or evaporation chamber into the gas flow stream.

Elements or steps described in relation to one embodiment may be combined with or replaced by elements or steps described in relation to another embodiment. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. Any reference signs in the claims should not be construed as limiting the scope.

**Claims**

1. An apparatus (100) for humidifying a gas flow stream (110), the apparatus comprising a plurality of humidification chambers (114, 116) configured to be connected in a sequence to allow the gas flow stream to flow sequentially through the plurality of humidification chambers, wherein each humidification chamber is configured to contain a liquid (124) over which the gas flow stream can flow and wherein at least one of the humidification chambers comprises a nozzle (118) configured to form the gas flow stream into a gas jet (120) that impinges on a surface (122) of the liquid in the humidification chamber.

2. The apparatus of claim 1, wherein the nozzle is configured to direct the gas jet towards the surface of the liquid such that, after impinging on the surface, the gas flow stream flows radially outwards from the point of impingement over the surface of the liquid to disrupt a boundary diffusion layer above the surface, to promote humidification of the gas flow stream.

3. The apparatus of claim 1 or 2, wherein the nozzle (118, 218) is configured to direct the gas jet (120, 220) towards the surface (122, 222) of the liquid (124, 224) such that a first velocity component (244) of a core (140, 240) of the gas jet in a direction perpendicular to the surface is greater than a second velocity component (246) of the core of the gas jet in a direction parallel to the surface.

4. The apparatus of any one of claims 1 to 3, wherein the nozzle is configured such that the gas jet impinges perpendicularly or substantially perpendicularly on the surface.

5. The apparatus of any preceding claim, comprising a thermally insulating portion configured to substantially prevent conduction of heat between adjacent humidification chambers.

6. The apparatus of any preceding claim, wherein at least one of the plurality of humidification chambers comprises a heat conductive wall (152) to facilitate extraction of heat from an external environment (154) of the at least one humidification chamber into the at least one humidification chamber to heat the liquid.

7. The apparatus of any one of claims 1 to 6, comprising an exterior wall (356) defining a passage (358) between the heat conductive wall (352) of at least one of the plurality of humidification chambers and the exterior wall (356), wherein the passage (358) is provided with an exhaust gas inlet (359) for receiving an exhaust gas stream (360) from a source device (350) for the gas flow stream to pass around an outside of the heat conductive wall (352) so that heat in the exhaust gas stream (360) conducts into the liquid in the humidification chamber.

8. The apparatus (100, 300) of any preceding claim, wherein a first humidification chamber (114, 314) in the sequence is configured to receive, at a gas inlet (126, 326) of the apparatus, the gas flow stream (110, 310) from a source device (350) of the gas flow stream such that the gas flow stream is passed through the first humidification chamber to increase a humidity level of the gas flow stream, and wherein a final humidification chamber (116, 316) in the sequence is configured to deliver a humidified gas flow stream (112, 312) to a gas outlet (134, 334) of the apparatus that has passed through each of the plurality of humidification chambers.

9. The apparatus of claim 8, wherein an intermediate humidification chamber (315) in the sequence is configured to receive the gas flow stream that has previously passed through the first humidification chamber such that the gas flow stream passes through the intermediate humidification chamber to increase the humidity of the gas flow stream and deliver, from the intermediate humidification chamber, the gas flow stream towards the final humidification chamber.

10. The apparatus of any preceding claim, wherein each humidification chamber comprises a nozzle configured to form the gas flow stream received by the humidification chamber into a gas jet (120) that impinges on a surface (122) of the liquid (124) in the humidification chamber.

11. The apparatus of any preceding claim, wherein the apparatus is a cold passover humidification apparatus.

12. A system (302) for humidifying a gas flow stream (310), comprising:

a source device (350) for generating the gas flow stream;
an apparatus (100, 300) according to any preceding claim; and
an interface (370) for delivering a humidified gas flow stream (312) from the apparatus to a subject.

13. A method of humidifying a gas flow stream (110), the method comprising:
passing a gas flow stream sequentially through a plurality of humidification chambers (114, 116) connected in a sequence, wherein each humidification chamber is configured to contain a liquid (124) over which the gas flow stream can flow and wherein the gas flow stream is formed into a gas jet (120) in at least one of the plurality of humidification chambers such that the gas jet impinges on a surface (122) of the liquid in the humidification chamber.

14. The method of claim 13, comprising:

   receiving, in a first humidification chamber (114, 314) in the sequence, the gas flow stream from a gas source, and passing the gas flow stream through the first humidification chamber to increase a humidity level of the gas flow stream; and

   delivering, from a final humidification chamber (116, 316) in the sequence, a humidified gas flow stream after passing the gas flow stream through the final humidification chamber to further increase the humidity level of the gas flow stream that has previously passed through the first humidification chamber.

15. The method of claim 13 or 14, comprising:
   passing the gas flow stream sequentially through the plurality of humidification chambers for a minimum period of time to decrease a temperature of the liquid in a first humidification chamber (114, 314) in the sequence to a temperature that is lower than a temperature of the liquid in a subsequent humidification chamber (116, 315, 316) in the sequence such that the temperature of the gas flow stream exiting the first humidification chamber is decreased compared with the temperature of the gas flow stream entering the first humidification chamber and such that the temperature of the gas flow stream passing through the subsequent humidification chamber is increased due to heat transfer from the liquid in the subsequent humidification chamber to the gas flow stream.

100

**Figure 1a**

126   114   128   130   132   116   134

110   118a   118b   112

120   122

124   154   152

118

120   142

140

122   110

124

**Figure 1b**

**Figure 2**

**Figure 3**

**Figure 4**

Figure 5

EP 3 785 755 A1

400

Receiving, in the first humidification chamber in the sequence, the gas flow stream from a gas source, and passing the gas flow stream through the first humidification chamber —402

Delivering, from the final humidification chamber in the sequence, a humidified gas flow stream after passing the gas flow stream through the final humidification chamber —404

Passing the gas flow stream sequentially through the plurality of humidification chambers for a minimum period of time to decrease a temperature of the liquid in a first humidification chamber in the sequence to a temperature that is lower than a temperature of the liquid in a subsequent humidification chamber in the sequence. —406

**Figure 6**

Europäisches
Patentamt

European
Patent Office

Office européen
des brevets

# EUROPEAN SEARCH REPORT

Application Number

EP 19 19 4097

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2015/167347 A1 (FISHER & PAYKEL HEALTHCARE LTD [NZ]) 5 November 2015 (2015-11-05) | 1-6,8-15 | INV. A61M16/16 A61M16/00 |
| Y | * paragraph [0090] - paragraph [0091] * * paragraph [0092] * * paragraph [0095] - paragraph [0096] * * paragraph [0099] * * paragraph [0103] * * paragraph [0105] * ----- | 7 | |
| Y | WO 2019/111110 A1 (FISHER & PAYKEL HEALTHCARE LTD [NZ]) 13 June 2019 (2019-06-13) * figure 12 * ----- | 7 | |
| A | US 2002/020930 A1 (AUSTIN GARY [US] ET AL) 21 February 2002 (2002-02-21) * figures * ----- | 1-15 | |
| A | US 2010/132708 A1 (MARTIN DION CHARLES CHEWE [AU] ET AL) 3 June 2010 (2010-06-03) * figure 10C * ----- | 1 | TECHNICAL FIELDS SEARCHED (IPC) A61M |
| E | WO 2019/190332 A1 (FISHER & PAYKEL HEALTHCARE LTD [NZ] ET AL.) 3 October 2019 (2019-10-03) * paragraph [00172]; figures 3A,11 * * paragraph [00180] * * paragraph [00157] * ----- | 1-15 | |

The present search report has been drawn up for all claims

| Place of search | Date of completion of the search | Examiner |
|---|---|---|
| Munich | 14 February 2020 | Valfort, Cyril |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 19 4097

This annex lists the patent family members relating to the patent documents cited in the above-mentioned European search report.
The members are as contained in the European Patent Office EDP file on
The European Patent Office is in no way liable for these particulars which are merely given for the purpose of information.

14-02-2020

| Patent document cited in search report | | Publication date | Patent family member(s) | | Publication date |
|---|---|---|---|---|---|
| WO 2015167347 | A1 | 05-11-2015 | US<br>WO | 2017035985 A1<br>2015167347 A1 | 09-02-2017<br>05-11-2015 |
| WO 2019111110 | A1 | 13-06-2019 | NONE | | |
| US 2002020930 | A1 | 21-02-2002 | AU<br>US<br>WO | 8717601 A<br>2002020930 A1<br>0213898 A2 | 25-02-2002<br>21-02-2002<br>21-02-2002 |
| US 2010132708 | A1 | 03-06-2010 | US<br>US<br>US<br>US | 2010132708 A1<br>2013247911 A1<br>2015190605 A1<br>2019247609 A1 | 03-06-2010<br>26-09-2013<br>09-07-2015<br>15-08-2019 |
| WO 2019190332 | A1 | 03-10-2019 | NONE | | |

EPO FORM P0459

For more details about this annex : see Official Journal of the European Patent Office, No. 12/82